# EUROPEAN PATENT APPLICATION

(11) **EP 3 928 815 A1**
(43) Date of publication of application: **29.12.2021**
(21) Application number: 20305683.3
(22) Date of filing: 22.06.2020
(51) Int. Cl.: A61M 5/315

(54) **SENSOR ASSEMBLY AND SYSTEM**

(71) Applicant: Becton Dickinson France, 38800 Le Pont-de-Claix (FR)
(72) Inventor: MARÉCHAL, Damien, 38640 Flaix (FR); GAGLIANO, Julien, 38000 GRENOBLE (FR); LE GAL-REDON, Patrick, 38170 SEYSSINET PARISET (FR)
(74) Representative: Germain Maureau

(57) **Abstract**

A sensor assembly for an injection device may include a housing and/or a mechanical interface. The housing may include a sensor and/or a wireless communication device. The sensor may be configured to detect a movement of a component of the injection device associated with an injection operation of the injection device. The wireless communication device may be configured to communicate, to a computing device, information associated with the movement of the component of the injection device. The mechanical interface may be configured to attach the housing to an exterior surface of the injection device. A system may include the sensor assembly and the injection device.

## Description

### BACKGROUND

### 1. Field

The present disclosure relates generally to a sensor assembly and, in some non-limiting embodiments or aspects, a sensory assembly for attachment to an injection device and configured for wireless communication.

### 2. Technical Considerations

A wide variety of injection devices for fluid injections are currently commercially available. However, many of these devices do not provide any mechanism to capture and/or transfer drug injection related data.

### SUMMARY

Accordingly, provided are improved systems, devices, products, apparatus, and/or methods for identifying devices connected to device connectors.

According to some non-limiting embodiments or aspects, provided is a sensor assembly for an injection device including: a housing including: a sensor configured to detect a movement of a component of the injection device associated with an injection operation of the injection device; and a wireless communication device configured to communicate, to a computing device, information associated with the movement of the component of the injection device; and a mechanical interface configured to attach the housing to an exterior surface of the injection device.

According to some non-limiting embodiments or aspects, provided is a system including: an injection device and a sensor assembly including: a housing including: a sensor configured to detect a movement of a component of the injection device associated with an injection operation of the injection device; and a wireless communication device configured to communicate, to a computing device, information associated with the movement of the component of the injection device; and a mechanical interface configured to attach the housing to an exterior surface of the injection device.

Further embodiments or aspects are set forth in the following numbered clauses:
Clause 1. A sensor assembly for an injection device comprising: a housing including: a sensor configured to detect a movement of a component of the injection device associated with an injection operation of the injection device; and a wireless communication device configured to communicate, to a computing device, information associated with the movement of the component of the injection device; and a mechanical interface configured to attach the housing to an exterior surface of the injection device.
Clause 2. The sensor assembly of clause 1, wherein the component of the injection device includes at least one of the following: a stopper of a plunger, a rod of the plunger, a mobile trigger that triggers the movement of the plunger, a mobile component of the injection device that triggers the injection operation, a needle shield, or any combination thereof, and wherein the sensor is configured to detect the movement of the at least one of the stopper of the plunger, the rod of the plunger, the mobile trigger, the mobile component of the injection device that triggers the injection operation, the needle shield, or any combination thereof.
Clause 3. The sensor assembly of any of clauses 1 and 2, wherein the information associated with the movement of the component of the injection device includes at least one of the following: a start time of the injection operation, a duration of the injection operation, an end time of the injection operation, a speed of the injection operation, a back pressure profile of the injection operation, a time of a needle shield deployment, a measured internal noise or vibration of the injection device (e.g., an acoustic and/or vibration profile, etc.), a measured injection force (e.g., a spring force, etc.), or any combination thereof.
Clause 4. The sensor assembly of any of clauses 1-3, wherein the housing includes one or more processors programmed and/or configured to determine, based on sensor data received from the sensor, the information associated with the movement of the component of the injection device.
Clause 5. The sensor assembly of any of clauses 1-4, wherein the housing includes a user feedback device, and wherein the user feedback device includes at least one of the following: a display, a light-emitting diode (LED), an audio output device, or any combination thereof.
Clause 6.The sensor assembly of any of clauses 1-5, wherein the housing includes a battery configured to power the sensor.
Clause 7. The sensor assembly of any of clauses 1-6, wherein the wireless communication device includes a short range wireless communication device.
Clause 8. The sensor assembly of any of clauses 1-7, wherein the housing includes a memory.
Clause 9. The sensor assembly of any of clauses 1-8, wherein the injection device includes one of the following: an auto injector, a pen injector, a wearable injector, a safety syringe device, a pre-filled syringe, and a passive needle guard.
Clause 10. The sensor assembly of any of clauses 1-9, wherein the mechanical interface is configured to index a position of the sensor relative to a drug inspection window of the injection device, and wherein the sensor is calibrated according to the indexed position of the sensor relative to the drug inspection window of the injection device.
Clause 11. The sensor assembly of any of clauses 1-10, wherein the housing is removably attached to the mechanical interface.
Clause 12. The sensor assembly of any of clauses 1-11, wherein the housing is integrated with the mechanical interface.
Clause 13. The sensor assembly of any of clauses 1-12, wherein the mechanical interface includes a base plate configured to be attached to the exterior surface of the injection device.
Clause 14. The sensor assembly of any of clauses 1-13, wherein the mechanical interface includes the exterior surface of the injection device.
Clause 15. The sensor assembly of any of clauses 1-14, wherein the mechanical interface includes an adhesive.
Clause 16. The sensor assembly of any of clauses 1-15, wherein the mechanical interface includes an adhesive label.
Clause 17. The sensor assembly of any of clauses 1-16, wherein the mechanical interface includes a ring or sleeve configured to fit around the exterior surface of the injection device, and wherein the ring or sleeve includes at least one of an elastomeric material and a heat-shrinkable material.
Clause 18. The sensor assembly of any of clauses 1-17, wherein the mechanical interface includes a rigid deformable clip configured to clip onto the exterior surface of the injection device.
Clause 19. The sensor assembly of any of clauses 1-18, wherein the sensor includes an optical sensor.
Clause 20. The sensor assembly of any of clauses 1-19, wherein, when the housing is attached to the injection device via the mechanical interface, the optical sensor is positioned to detect the movement of the component of the injection device through a drug inspection window of the injection device.
Clause 21. The sensor assembly of any of clauses 1-20, wherein the optical sensor includes an array of photodetector and LED pairs.
Clause 22. The sensor assembly of any of clauses 1-21, wherein the optical sensor includes a single photodetector and LED pair, and wherein the component of the injection device includes a printed or gradated pattern visible through the drug inspection window of the injection device.
Clause 23. The sensor assembly of any of clauses 1-22, wherein, when the housing is attached to the injection device via the mechanical interface, the housing partially overlaps a drug inspection window of the injection device.
Clause 24. The sensor assembly of any of clauses 1-23, wherein the injection device includes a plurality of drug inspection windows, and wherein, when the housing is attached to the injection device via the mechanical interface, the housing fully overlaps one of the plurality of drug inspection windows.
Clause 25. The sensor assembly of any of clauses 1-24, wherein the sensor includes an acoustic or vibration sensor.
Clause 26. The sensor assembly of any of clauses 1-25, wherein, when the housing is attached to the injection device via the mechanical interface, the acoustic or vibration sensor is positioned to measure an internal noise or vibration of the injection device to detect the movement of the component of the injection device.
Clause 27. The sensor assembly of any of clauses 1-26, further comprising: one or more processors programmed and/or configured to compare a signal received from the acoustic sensor or the vibration sensor to one or more reference signals to determine at least one of the following: a start time of the injection operation, a duration of the injection operation, an end time of the injection operation, a speed of the injection operation, a back pressure profile of the injection operation, a time of a needle shield deployment, a measured internal noise or vibration of the injection device, a measured injection force, or any combination thereof.
Clause 28. The sensor assembly of any of clauses 1-27, wherein the sensor includes a magnetic sensor.
Clause 29. The sensor assembly of any of clauses 1-28, wherein the component of the injection device includes a magnet, and wherein, when the housing is attached to the injection device via the mechanical interface, the magnetic sensor is positioned to measure a magnetic field modification and/or a magnetic evolution created by the magnet to detect the movement of the component of the injection device.
Clause 30. The sensor assembly of any of clauses 1-29, wherein, when the housing is attached to the injection device via the mechanical interface, the magnetic sensor is positioned at a center of a range of motion of the magnet.
Clause 31. The sensor assembly of any of clauses 1-30, wherein the magnet includes a printed or gradated magnetic pattern or scale.
Clause 32. A system comprising: an injection device; and the sensor assembly according to any of claims 1-31.

These and other features and characteristics of the present disclosure, as well as the methods of operation and functions of the related elements of structures and the combination of parts and economies of manufacture, will become more apparent upon consideration of the following description and the appended claims with reference to the accompanying drawings, all of which form a part of this specification, wherein like reference numerals designate corresponding parts in the various figures. It is to be expressly understood, however, that the drawings are for the purpose of illustration and description only and are not intended as a definition of limits. As used in the specification and the claims, the singular form of "a," "an," and "the" include plural referents unless the context clearly dictates otherwise.

### BRIEF DESCRIPTION OF THE DRAWINGS

Additional advantages and details of embodiments or aspects of the present disclosure are explained in greater detail below with reference to the exemplary embodiments that are illustrated in the accompanying schematic figures, in which:
FIG. 1A is a diagram of non-limiting embodiments or aspects of an environment in which systems, devices, products, apparatus, and/or methods, described herein, may be implemented;
FIG. 1B is a perspective view of non-limiting embodiments or aspects of components of one or more devices and/or one or more systems of FIG. 1A;
FIG. 1C is a diagram of non-limiting embodiments or aspects of components of one or more devices and/or one or more systems of FIGS. 1A and 1 B;
FIG. 2 is a perspective view of an implementation of non-limiting embodiments or aspects of a mechanical interface;
FIG. 3 is a perspective view of an implementation of non-limiting embodiments or aspects of mechanical interface;
FIG. 4 is a perspective view of an implementation of non-limiting embodiments or aspects of a mechanical interface;
FIG. 5 is a perspective view of an implementation of non-limiting embodiments or aspects of a mechanical interface;
FIG. 6A is a diagram of an implementation of non-limiting embodiments or aspects of a sensor;
FIG. 6B is a diagram of an implementation of non-limiting embodiments or aspects of a sensor;
FIG. 7 is a diagram of an implementation of non-limiting embodiments or aspects of a sensor;
FIG. 8A is a diagram of an implementation of non-limiting embodiments or aspects of a sensor; and
FIG. 8B is a diagram of an implementation of non-limiting embodiments or aspects of a sensor.

### DETAILED DESCRIPTION

It is to be understood that the present disclosure may assume various alternative variations and step sequences, except where expressly specified to the contrary. It is also to be understood that the specific devices and processes illustrated in the attached drawings, and described in the following specification, are simply exemplary and non-limiting embodiments or aspects. Hence, specific dimensions and other physical characteristics related to the embodiments or aspects disclosed herein are not to be considered as limiting.

For purposes of the description hereinafter, the terms "end," "upper," "lower," "right," "left," "vertical," "horizontal," "top," "bottom," "lateral," "longitudinal," and derivatives thereof shall relate to the present disclosure as it is oriented in the drawing figures. However, it is to be understood that the present disclosure may assume various alternative variations and step sequences, except where expressly specified to the contrary. It is also to be understood that the specific devices and processes illustrated in the attached drawings, and described in the following specification, are simply exemplary embodiments or aspects of the present disclosure. Hence, specific dimensions and other physical characteristics related to the embodiments or aspects of the embodiments disclosed herein are not to be considered as limiting unless otherwise indicated.

No aspect, component, element, structure, act, step, function, instruction, and/or the like used herein should be construed as critical or essential unless explicitly described as such. Also, as used herein, the articles "a" and "an" are intended to include one or more items, and may be used interchangeably with "one or more" and "at least one." Furthermore, as used herein, the term "set" is intended to include one or more items (e.g., related items, unrelated items, a combination of related and unrelated items, etc.) and may be used interchangeably with "one or more" or "at least one." Where only one item is intended, the term "one" or similar language is used. Also, as used herein, the terms "has," "have," "having," or the like are intended to be open-ended terms. Further, the phrase "based on" is intended to mean "based at least in partially on" unless explicitly stated otherwise.

As used herein, the terms "communication" and "communicate" refer to the receipt or transfer of one or more signals, messages, commands, or other type of data. For one unit (e.g., any device, system, or component thereof) to be in communication with another unit means that the one unit is able to directly or indirectly receive data from and/or transmit data to the other unit. This may refer to a direct or indirect connection that is wired and/or wireless in nature. Additionally, two units may be in communication with each other even though the data transmitted may be modified, processed, relayed, and/or routed between the first and second unit. For example, a first unit may be in communication with a second unit even though the first unit passively receives data and does not actively transmit data to the second unit. As another example, a first unit may be in communication with a second unit if an intermediary unit processes data from one unit and transmits processed data to the second unit. It will be appreciated that numerous other arrangements are possible.

It will be apparent that systems and/or methods, described herein, can be implemented in different forms of hardware, software, or a combination of hardware and software. The actual specialized control hardware or software code used to implement these systems and/or methods is not limiting of the implementations. Thus, the operation and behavior of the systems and/or methods are described herein without reference to specific software code, it being understood that software and hardware can be designed to implement the systems and/or methods based on the description herein.

Some non-limiting embodiments or aspects are described herein in connection with thresholds. As used herein, satisfying a threshold may refer to a value being greater than the threshold, more than the threshold, higher than the threshold, greater than or equal to the threshold, less than the threshold, fewer than the threshold, lower than the threshold, less than or equal to the threshold, equal to the threshold, etc.

As used herein, the term "computing device" or "computer device" may refer to one or more electronic devices that are configured to directly or indirectly communicate with or over one or more networks. The computing device may be a mobile device, a desktop computer, or the like. Furthermore, the term "computer" may refer to any computing device that includes the necessary components to receive, process, and output data, and normally includes a display, a processor, a memory, an input device, and a network interface. An "application" or "application program interface" (API) refers to computer code or other data sorted on a computer-readable medium that may be executed by a processor to facilitate the interaction between software components, such as a client-side front-end and/or server-side back-end for receiving data from the client. An "interface" refers to a generated display, such as one or more graphical user interfaces (GUIs) with which a user may interact, either directly or indirectly (e.g., through a keyboard, mouse, touchscreen, etc.).

As used herein, the term "server" may refer to or include one or more processors or computers, storage devices, or similar computer arrangements that are operated by or facilitate communication and processing for multiple parties in a network environment, such as the Internet, although it will be appreciated that communication may be facilitated over one or more public or private network environments and that various other arrangements are possible. Further, multiple computers, e.g., servers, or other computerized devices, such as POS devices, directly or indirectly communicating in the network environment may constitute a "system," such as a merchant's POS system. As used herein, the term "data center" may include one or more servers, or other computing devices, and/or databases.

As used herein, the term "mobile device" may refer to one or more portable electronic devices configured to communicate with one or more networks. As an example, a mobile device may include a cellular phone (e.g., a smartphone or standard cellular phone), a portable computer (e.g., a tablet computer, a laptop computer, etc.), a wearable device (e.g., a watch, pair of glasses, lens, clothing, and/or the like), a personal digital assistant (PDA), and/or other like devices. The terms "client device" and "user device," as used herein, refer to any electronic device that is configured to communicate with one or more servers or remote devices and/or systems. A client device or user device may include a mobile device, a network-enabled appliance (e.g., a network-enabled television, refrigerator, thermostat, and/or the like), a computer, and/or any other device or system capable of communicating with a network.

As used herein, the term "application" or "application program interface" (API) refers to computer code, a set of rules, or other data sorted on a computer-readable medium that may be executed by a processor to facilitate interaction between software components, such as a client-side front-end and/or server-side back-end for receiving data from the client. An "interface" refers to a generated display, such as one or more graphical user interfaces (GUIs) with which a user may interact, either directly or indirectly (e.g., through a keyboard, mouse, etc.).

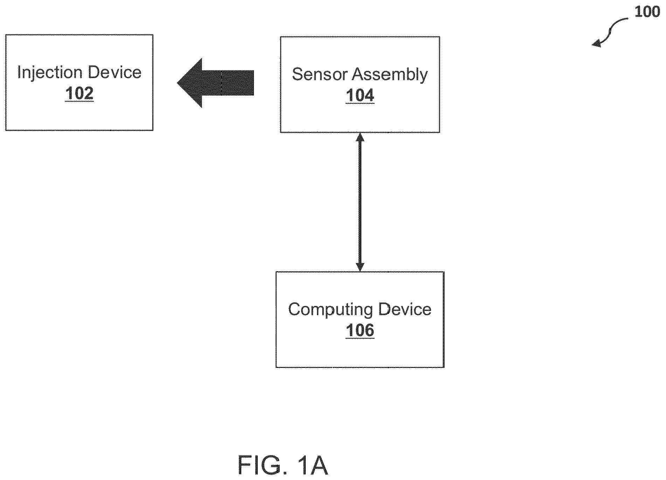

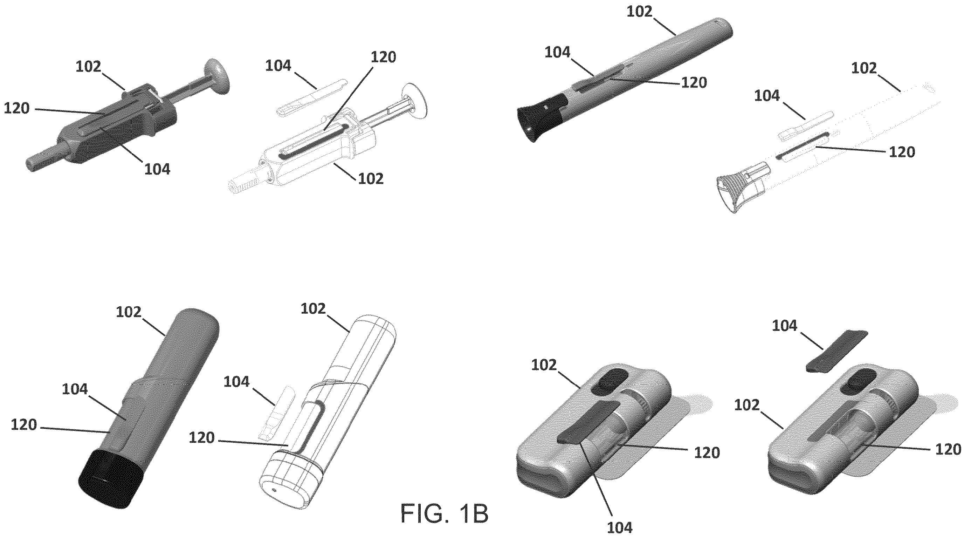

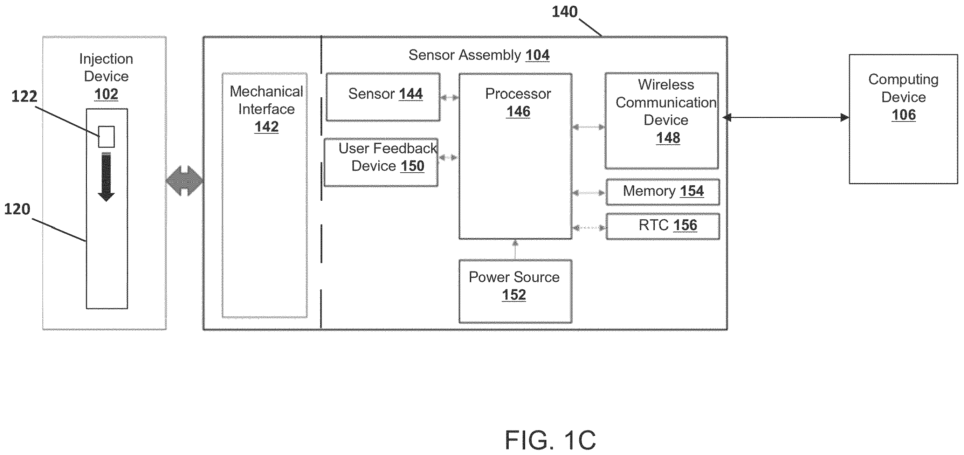

Referring to FIG. 1A, non-limiting embodiments or aspects of an environment 100 in which systems, devices, products, apparatus, and/or methods, as described herein, may be implemented is shown. As shown in FIG. 1, environment 100 may include injection device 102, sensor assembly 104, and/or computing device 106.

Injection device 102 may be configured to perform an injection operation to deliver a dose of medicament to a user. Injection device 102 may include injection devices with or without injection needles. Injection device 102 may be a disposable type injection device (e.g., a single use injection device, etc.) or a re-usable type injection device. Injection device 102 may include an auto injector, a pen injector, a wearable injector, and/or a passive needle guard. For example, injection device 102 may include the BD Intevia^{™} autoinjector, the BD Libertas^{™} wearable autoinjector, the BD Sonic^{™} autoinjector, and/or the BD UltraSafe Plus^{™} passive needle guard for a pre-filled ISO standard glass syringe. As an example, FIG. 1B shows various non-limiting embodiments or aspects of injection device 102.

Referring also to FIG. 1C, injection device 102 may include drug inspection window 120. For example, drug inspection window 120 may include a drug inspection window as per required in the ISO 11608 standard. As an example, drug inspection window 120 may be positioned and dimensioned with respect to injection device 102 as per required in the ISO 11608 standard. For example, FIG. 1B shows drug inspection window 120 as included in various non-limiting embodiments or aspects of injection device 102. In some non-limiting embodiments or aspects, injection device 102 may include a needle shield that is automatically deployed (e.g., at an end time of an injection operation, etc.) or manually deployed (e.g., by a user, etc.).

Still referring to FIG. 1C, component 122 of injection device 102 may be configured for movement in association with an injection operation of injection device 102. For example, component 122 may include a plunger stopper, a plunger rod, a needle shield of injection device 102, and/or a mobile trigger or component that initiates or triggers the injection operation (e.g., a button, a trigger, and/or the like that moves or is displaced to initiate and/or perform the injection operation to deliver a dose of a medicament to a user, etc.).

Sensor assembly 104 may include a housing 140 and a mechanical interface 142. Housing 140 may include (e.g., house, encompass, etc.) a sensor 144, a processor 146, a wireless communication device 148, a user feedback device 150, a power source 152, a memory 154, and/or a real-time clock (RTC) 156.

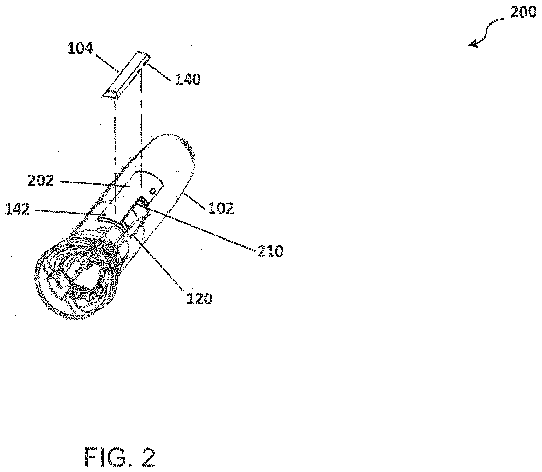

Mechanical interface 142 may be configured to attach (e.g., removably attach, permanently attach, etc.) housing 140 to an exterior surface of injection device 102. For example, housing 140 may be removably attached to mechanical interface 142, permanently attached to mechanical interface 142, and/or integrated with mechanical interface 142. In some non-limiting embodiments or aspects, mechanical interface 142 is configured to index a position of sensor 144 relative to drug inspection window 120 of injection device 102 and/or component 122 within injection device 102. For example, sensor 144 may be calibrated according to the indexed position of the sensor relative to drug inspection window 120 of injection device 102 and/or a position of component 122 within injection device 102. As an example, a first mechanical interface 142 may be configured to index a position of sensor 144 relative to drug inspection window 120 of injection device 102 and/or component 122 within injection device 102 for a first type of injection device 102, and a second mechanical interface 142 may be configured to index a position of sensor 144 relative to drug inspection window 120 of injection device 102 and/or component 122 within injection device 102 for a second type of injection device 102 different than the first type of injection device. Further details regarding non-limiting embodiments or aspects of mechanical interface 142 are provided below with regard to FIGS. 2-5.

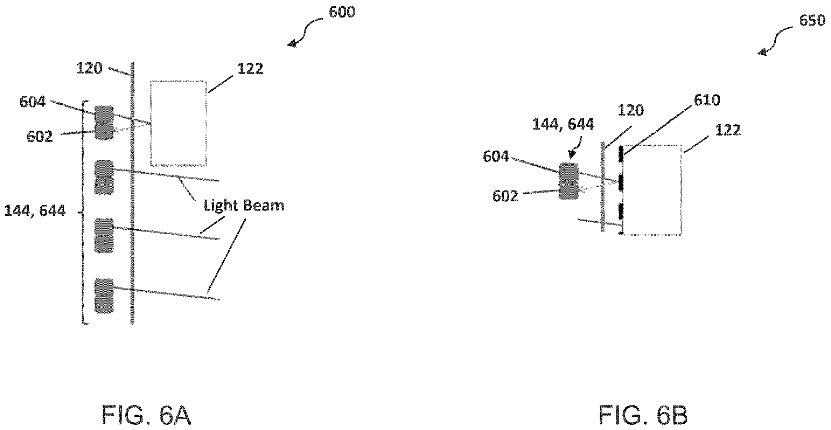

Sensor 144 may be configured to detect a movement of component 122 of injection device 102 associated with an injection operation of injection device 102. For example, sensor 144 may be configured to detect a movement (e.g., a movement, a displacement, etc.) of at least one of a piston, a stopper of a plunger, a rod of the plunger, and a mobile trigger that initiates or triggers the movement of the plunger. In some non-limiting embodiments or aspects, sensor 144 may include at least one of the following types of sensors: an optical sensor, an acoustic sensor, a vibration sensor, an acceleration sensor, a magnetic sensor, or any combination thereof. Further details regarding non-limiting embodiments or aspects of sensor 144 are provided below with regard to FIGS. 6A, 6B, 7, 8A, and 8B.

Processor 146 may be programmed and/or configured to determine, based on a signal or sensor data received from sensor 144, information associated with a movement of component 122 of injection device 102. In some non-limiting embodiments or aspects, processor 146 may include a low power microcontroller unit (MCU). In some non-limiting embodiments or aspects, information associated with movement of component 122 of injection device 102 includes at least one of the following: a start time of the injection operation, a duration of the injection operation, an end time of the injection operation, a speed of the injection operation, a back pressure profile of the injection operation, a time of a needle shield deployment, a measured internal noise or vibration of injection device 102 (e.g., an acoustic and/or vibration profile, etc.), a measured injection force (e.g., a spring force, etc.) or any combination thereof. Processor 146 may store information associated with movement of component 122 of injection device 102 in memory 154.

Wireless communication device 148 may be configured to wirelessly communicate with computing device 106. For example, wireless communication device 148 may be configured to communicate information associated with movement of component 122 of injection device 102 to computing device 106. In some non-limiting embodiments or aspects, wireless communication device 148 includes one or more computing devices, chips, contactless transmitters, contactless transceivers, NFC transmitters, RFID transmitters, contact based transmitters, Bluetooth transceivers^{®} and/or the like that enables wireless communication device 148 to receive information directly from and/or communicate information directly to computing device 106 via a short range wireless communication connection (e.g., a communication connection that uses NFC protocol, a communication connection that uses Radio-frequency identification (RFID), a communication connection that uses a Bluetooth^{®} wireless technology standard, and/or the like).

User feedback device 150 may be configured to provide an indication associated with an injection operation and/or information associated with movement of component 122 of injection device 102 to a user. For example, user feedback device 150 may include at least one of the following: a display, a light-emitting diode (LED), an audio output device (e.g., a buzzer, a speaker, etc.), a tactile or haptic feedback device (e.g., a vibrator, etc.) or any combination thereof.

Power source 152 may be configured to power sensor 144, processor 146, wireless communication device 148, user feedback device 150, memory 154, and/or RTC 156. For example, power source 152 may include a battery (e.g., a rechargeable battery, a disposable battery, etc.), an energy harvester (e.g., an energy harvester configured to derive energy from one or more external sources, such as electromagnetic energy, solar energy, thermal energy, wind energy, salinity gradients, kinetic energy, and/or the like, etc.), or any combination thereof.

Memory 154 may be configured to store information associated with movement of component 122 of injection device 102 in memory 154. In some non-limiting embodiments or aspects, memory 154 may store calibration data associated with one or more calibration settings of sensor 144. For example, sensor 144 may be according to an indexed position of sensor 144 relative to drug inspection window 120 of injection device 102. As an example, mechanical interface 142 may be configured to index a position of sensor 144 relative to drug inspection window 120 of injection device 102.

RTC 156 may include a computer clock (e.g., in the form of an integrated circuit, etc.) that keeps track of the current time. For example, processor 146 may time stamp information associated with a movement of component 122 of injection device 102 based on the current time of RTC 156.

Computing device 106 may include one or more devices capable of receiving information and/or data from sensor assembly 104 and/or one or more other computing devices and/or communicating information and/or data to sensor assembly 104 and/or the one or more other computing devices. For example, computing device 106 may include a computing device, a server, a group of servers, a mobile device, a group of mobile devices, a receiving unit, a router device, a bridge device, a hub device, and/or the like. In some non-limiting embodiments or aspects, computing device 106 includes one or more computing devices, chips, contactless transmitters, contactless transceivers, NFC transmitters, RFID transmitters, contact based transmitters, and/or the like that enables computing device 106 to receive information directly from and/or communicate information directly to wireless communication device 148 via a short range wireless communication connection (e.g., a communication connection that uses NFC protocol, a communication connection that uses Radio-frequency identification (RFID), a communication connection that uses a Bluetooth^{®} wireless technology standard, and/or the like). In some non-limiting embodiments or aspects, computing device 106 may include and/or upload information and/or data to an electronic data management system, such as a hospital record system, a system used during a clinical trial to collect the trial related information, and/or the like.

The number and arrangement of devices and systems shown in FIGS. 1A-1C is provided as an example. There may be additional devices and/or systems, fewer devices and/or systems, different devices and/or systems, or differently arranged devices and/or systems than those shown in FIGS. 1A-1C. Furthermore, two or more devices and/or systems shown in FIGS. 1A-1C may be implemented within a single device and/or system, or a single device and/or system shown in FIGS. 1A-1C may be implemented as multiple, distributed devices and/or systems. Additionally, or alternatively, a set of devices and/or systems (e.g., one or more devices or systems) of environment 100 may perform one or more functions described as being performed by another set of devices and/or systems of environment 100.

Referring now to FIG. 2, FIG. 2 is a perspective view of an implementation 200 of non-limiting embodiments or aspects of mechanical interface 142. As shown in FIG. 2, mechanical interface 142 may include base plate 202. Base plate 202 may be configured to be attached to an exterior surface of injection device 102. For example, base plate 202 may be attached to an exterior surface of injection device 102 via a clip, a snap fit, an adhesive, and/or other mechanical interface. Base plate 202 may be a rigid plate or base or a flexible plate or base. Sensor assembly 104 may be attached to and/or integrated with base plate 202. For example, sensor assembly 104 may be attached to base plate 202 via a clip, a snap fit, an adhesive, and/or other mechanical interface. Base plate 202 may be configured and/or dimensioned according to a type of injection device 102. For example, base plate 202 may be configured to index a position of sensor 144 relative to drug inspection window 120 and/or component 122 of injection device 120, with sensor 144 being calibrated according to the indexed position. As further shown in FIG. 2, base plate 202 may include a cutout or opening 210 corresponding to a portion of drug inspection window 120 of injection device 102. Cutout or opening 210 may be sized and configured to leave a portion of drug inspection window 102 visible to a user when base plate 202 and/or sensor assembly 104 is attached to injection device 102. In some non-limiting embodiments or aspects, cutout or opening 210 may provide an indication of a position and orientation at which to attach base plate 202 to injection device 102 with respect to drug inspection window 120 of injection device 102. In some non-limiting embodiments or aspects, injection device 102 may include one or more markings, patterns, recesses, holes, and/or protrusions that provide an indication of a position and orientation at which to attach base plate 202 to injection device 102. In some non-limiting embodiments or aspects, base plate 202 may include one or more corresponding or complementary markings, patterns, recesses, holes, and/or protrusions corresponding to the one or more markings, patterns, recesses, holes, and/or protrusions on injection device 102.

In some non-limiting embodiments or aspects, base plate 202 may include an external feature (e.g., a clamshell, etc.), and sensor assembly 104 may be integrated in the external feature (e.g., within the clamshell, etc.)

In some non-limiting embodiments or aspects, base plate 202 may include an external surface of injection device 102. For example, base plate 202 may be integrated with the external surface of injection device 202.

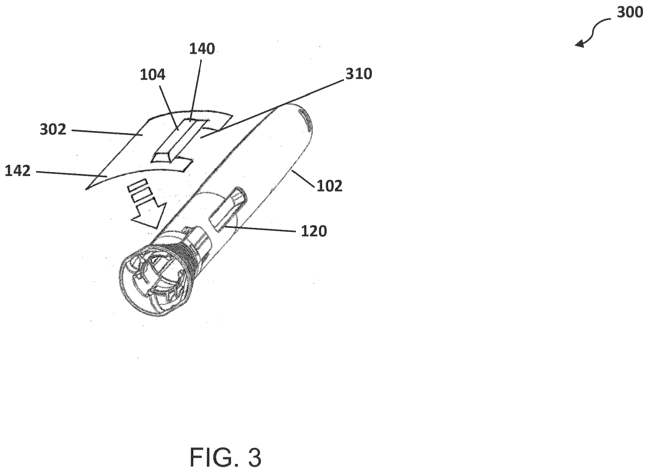

Referring now to FIG. 3, FIG. 3 is a perspective view of an implementation 300 of non-limiting embodiments or aspects of mechanical interface 142. As shown in FIG. 2, mechanical interface 142 may include adhesive label 302. Adhesive label may be configured to be attached to an exterior surface of injection device 102 via an adhesive one a bottom surface of adhesive label 302. Adhesive label 302 may include a flexible label or base. Sensor assembly 104 may be attached to (e.g., via an adhesive on a top surface opposite the bottom surface, etc.) and/or integrated with adhesive label 302. Adhesive label 302 may be configured and/or dimensioned according to a type of injection device 102. For example, adhesive label 302 may be configured to index a position of sensor 144 relative to drug inspection window 120 and/or component 122 of injection device 120, with sensor 144 being calibrated according to the indexed position. As further shown in FIG. 3, adhesive label 302 may include a cutout or opening 310 corresponding to a portion of drug inspection window 120 of injection device 102. Cutout or opening 310 may be sized and configured to leave a portion of drug inspection window 102 visible to a user when adhesive label 302 and/or sensor assembly 104 is attached to injection device 102. In some non-limiting embodiments or aspects, cutout or opening 310 may provide an indication of a position and orientation at which to attach adhesive label 302 to injection device 102 with respect to drug inspection window 120 of injection device 102. In some non-limiting embodiments or aspects, injection device 102 may include one or more markings, patterns, recesses, holes, and/or protrusions that provide an indication of a position and orientation at which to attach adhesive label 302 to injection device 102. In some non-limiting embodiments or aspects, adhesive label 302 may include one or more corresponding or complementary markings, patterns, recesses, holes, and/or protrusions corresponding to the one or more markings, patterns, recesses, holes, and/or protrusions on injection device 102.

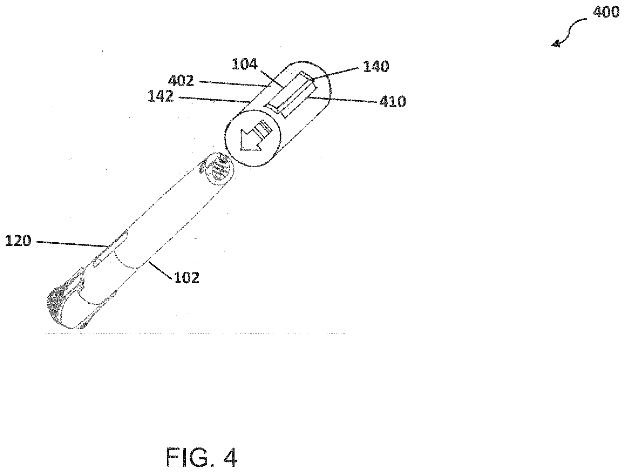

Referring now to FIG. 4, FIG. 4 is a perspective view of an implementation 400 of non-limiting embodiments or aspects of mechanical interface 142. As shown in FIG. 2, mechanical interface 142 may include ring or sleeve 402. Ring or sleeve 402 may be configured to fit around an exterior surface (e.g., an exterior circumference, etc.) of injection device 102. Ring or sleeve 402 may include at least one of an elastomeric material and a heat-shrinkable material. Sensor assembly 104 may be attached to and/or integrated with ring or sleeve 402. For example, sensor assembly 104 may be attached to ring or sleeve 402 via a clip, a snap fit, an adhesive, and/or other mechanical interface. Ring or sleeve 402 may be configured and/or dimensioned according to a type of injection device 102. For example, ring or sleeve 402 may be configured to index a position of sensor 144 relative to drug inspection window 120 and/or component 122 of injection device 120, with sensor 144 being calibrated according to the indexed position. As further shown in FIG. 4, ring or sleeve 402 may include a cutout or opening 410 corresponding to a portion of drug inspection window 120 of injection device 102. Cutout or opening 410 may be sized and configured to leave a portion of drug inspection window 102 visible to a user when ring or sleeve 402 and/or sensor assembly 104 is attached to injection device 102. In some non-limiting embodiments or aspects, cutout or opening 410 may provide an indication of a position and orientation at which to attach ring or sleeve 402 to injection device 102 with respect to drug inspection window 120 of injection device 102. In some non-limiting embodiments or aspects, injection device 102 may include one or more markings, patterns, recesses, holes, and/or protrusions that provide an indication of a position and orientation at which to attach ring or sleeve 402 to injection device 102. In some non-limiting embodiments or aspects, ring or sleeve 402 may include one or more corresponding or complementary markings, patterns, recesses, holes, and/or protrusions corresponding to the one or more markings, patterns, recesses, holes, and/or protrusions on injection device 102.

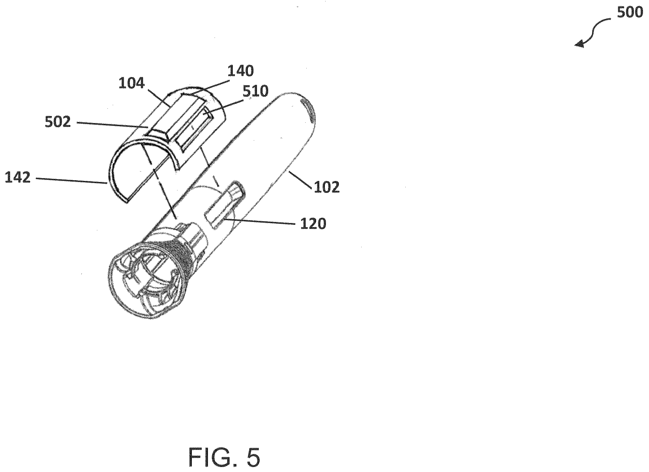

Referring now to FIG. 5, FIG. 5 is a perspective view of an implementation 500 of non-limiting embodiments or aspects of mechanical interface 142. As shown in FIG. 2, mechanical interface 142 may include a rigid deformable clip 502. Rigid deformable clip 502 may be configured to be clip onto an exterior surface of injection device 102. For example, rigid deformable clip 502 may include a half-pipe shape, with a material forming the half-pipe shape being configured to deform around and grasp an exterior surface of injection device 102 when clip 502 is pressed onto injection device 102. Sensor assembly 104 may be attached to and/or integrated with rigid deformable clip 502. For example, sensor assembly 104 may be attached to rigid deformable clip 502 via a clip, a snap fit, an adhesive, and/or other mechanical interface. Rigid deformable clip 502 may be configured and/or dimensioned according to a type of injection device 102. For example, rigid deformable clip 502 may be configured to index a position of sensor 144 relative to drug inspection window 120 and/or component 122 of injection device 120, with sensor 144 being calibrated according to the indexed position. As further shown in FIG. 5, rigid deformable clip 502 may include a cutout or opening 510 corresponding to a portion of drug inspection window 120 of injection device 102. Cutout or opening 210 may be sized and configured to leave a portion of drug inspection window 102 visible to a user when rigid deformable clip 502 and/or sensor assembly 104 is attached to injection device 102. In some non-limiting embodiments or aspects, cutout or opening 510 may provide an indication of a position and orientation at which to attach rigid deformable clip 502 to injection device 102 with respect to drug inspection window 120 of injection device 102.

Referring now to FIGS. 6A and 6B, FIGS. 6A and 6B are diagrams of implementations 600 and 650 of non-limiting embodiments or aspects of sensor 144. As shown in FIGS. 6A and 6B, sensor 144 may include optical sensor 644. For example, when housing 140 is attached to injection device 102 via mechanical interface 142, optical sensor 644 is positioned to detect a movement of component 122 of injection device 102 through drug inspection window 120 of injection device 102. As an example, optical sensor 644 may be positioned adjacent and/or over drug inspection window 102 to directly measure or capture a movement component 122 of injection device 102 through drug inspection window 120. In some non-limiting embodiments or aspects, when housing 140 is attached to injection device 102 via mechanical interface 142, housing 140 partially overlaps drug inspection window 120 of injection device 102. In some non-limiting embodiments or aspects, injection device 102 includes a plurality of drug inspection windows 120 and, when housing 140 is attached to injection device 102 via mechanical interface 142, housing 140 fully overlaps or covers (e.g., complete obscures a view of, etc.) one of the plurality of drug inspection windows 120.

As further shown in FIG. 6A, in some non-limiting embodiments or aspects, optical sensor 644 includes an array of photodetector 602 and LED 604 pairs. For example, photodetector 602 and LED 604 pairs may be aligned along drug inspection window 120. Each LED 604 may transmit light beams through drug inspection window 120, which may be reflected by component 122 when component 122 is moved in front of that LED 604. Photodetector 602 corresponding to that LED 604 may detect the reflected light beam, while other photodetectors 602 may not detect light beams which are not reflected by component 122 when component 122 is not in front of a corresponding photodetector 602 and LED 604 pair. Processor 146 may drive LEDs 604 and/or receive acquisition signals from photodetectors 602. Based on the received acquisition signals, processor 146 may perform signal processing (e.g., filtering, pattern detection etc.) on the received signals, perform threshold level(s) extraction, and/or determine information associated with a movement of component 122 of injection 102.

As further shown in FIG. 6B, in some non-limiting embodiments or aspects, optical sensor 644 includes a single photodetector 602 and LED 604 pair, and component 122 of injection device102 includes a printed or gradated pattern 610 visible through drug inspection window 120 of injection device 102. LED 602 may transmit light beams through drug inspection window 120, which may be reflected by pattern 610 when component 122 is in front of and/or moved past LED 604. Photodetector 602 may detect the reflected light beams, the reflection of which may change according to a position of pattern 610 with respect to photodetector 602 and/or LED 604. Processor 146 may be programmed and/or configured to drive LED 604 and/or receive acquisition signals from photodetector 602. Based on the received acquisition signals, processor 146 may be programmed and/or configured to perform signal processing (e.g., filtering, pattern detection etc.) on the received signals, perform threshold level(s) extraction, and/or determine information associated with a movement of component 122 of injection 102.

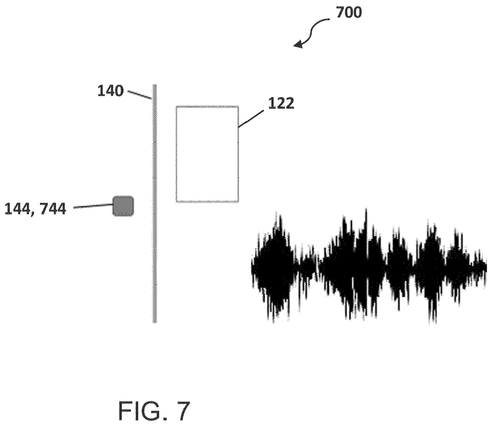

Referring now to FIG. 7, FIG. 7 is a diagram of an implementation 700 of non-limiting embodiments or aspects of sensor 144. As shown in FIG. 7, sensor 144 may include acoustic or vibration sensor 744. For example, when housing 140 is attached to injection device 102 via mechanical interface 142, acoustic or vibration sensor 744 may be positioned to measure a noise or vibration (e.g., an internal noise or vibration, a spring movement, an internal shock, an end of dose indicator, such as an audible click, and/or the like, etc.) of injection device 102 to detect a movement of component 122 of injection device 102. As an example, acoustic or vibration sensor 744 may be positioned adjacent an exterior surface of injection device 102.

Processor 146 may be programmed and/or configured to receive acquisition signals from acoustic or vibration sensor 744. Based on the received acquisition signals, processor 146 may be programmed and/or configured to perform signal processing (e.g., filtering, pattern detection etc.) on the received signals, perform threshold level(s) extraction, and/or determine information associated with a movement of component 122 of injection 102. For example, processor 146 may be programmed and/or configured to compare a signal received from acoustic or vibration sensor 744 to one or more reference signals (e.g., stored in memory 154, etc.) to determine at least one of the following: a start time of the injection operation, a duration of the injection operation, a speed of the injection operation, a back pressure profile of the injection operation, or any combination thereof.

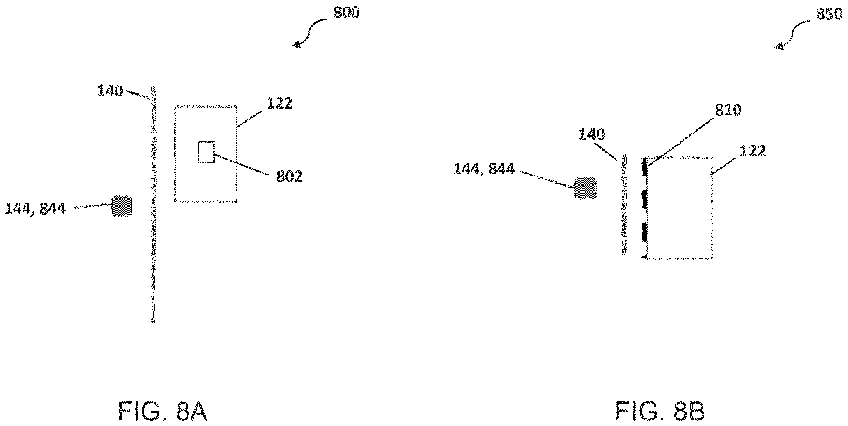

Referring now to FIGS. 8A and 8B, FIGS. 8A and 8B are diagrams of implementations 800 and 850 of non-limiting embodiments or aspects of sensor 144. As shown in FIGS. 8A and 8B, sensor 144 may include magnetic sensor 844 (e.g., a Hall effect sensor, a linear Hall effect sensor, a latch Hall effect sensors, a reed relay, etc.). In such an example, component 122 of injection device 102 may include magnet 802 (e.g., a magnet attached to component 122, a magnetic coating, component 122 formed of a magnetic material, etc.). For example, when housing 140 is attached to injection device 102 via mechanical interface 142, magnetic sensor 844 is positioned to measure a magnetic field modification and/or a magnetic evolution created by magnet 802 included in and/or on component 122 to detect a movement of component 122 of injection device 102. As an example, magnetic sensor 844 may be positioned adjacent an exterior surface of injection device 102.

As further shown in FIG. 8A, in some non-limiting embodiments or aspects, when housing 140 is attached to injection device 102 via mechanical interface 142, magnetic sensor 844 is positioned at a center of a range of motion of magnet 802 on and/or in component 122. For example, if magnet 802 is configured to travel a distance D between a first point and a second point during an injection operation of injection device 102, magnetic sensor 844 may be positioned at a distance D/2 halfway between the first point and the second point in a direction of travel of the magnet 802.

As further shown in FIG. 8B, in some non-limiting embodiments or aspects, magnet 802 may include printed or gradated magnetic pattern or scale 810 on and/or integrated with component 122. For example, printed or gradated magnetic pattern or scale 810 may ease a movement detection of component 122 and/or enable magnetic sensor 844 to be located in a more diverse array or locations with respect to component 122 in injection device 120 when housing 140 is attached to injection device 102 via mechanical interface 142.

Although embodiments or aspects have been described in detail for the purpose of illustration and description, it is to be understood that such detail is solely for that purpose and that embodiments or aspects are not limited to the disclosed embodiments or aspects, but, on the contrary, are intended to cover modifications and equivalent arrangements that are within the spirit and scope of the appended claims. For example, it is to be understood that the present disclosure contemplates that, to the extent possible, one or more features of any embodiment or aspect can be combined with one or more features of any other embodiment or aspect. In fact, any of these features can be combined in ways not specifically recited in the claims and/or disclosed in the specification. Although each dependent claim listed below may directly depend on only one claim, the disclosure of possible implementations includes each dependent claim in combination with every other claim in the claim set.

## Claims

1. A sensor assembly for an injection device comprising:
a housing including:
a sensor configured to detect a movement of a component of the injection device associated with an injection operation of the injection device; and
a wireless communication device configured to communicate, to a computing device, information associated with the movement of the component of the injection device; and
a mechanical interface configured to attach the housing to an exterior surface of the injection device.

2. The sensor assembly of claim 1, wherein the component of the injection device includes at least one of the following: a stopper of a plunger, a rod of the plunger, a mobile trigger that triggers the movement of the plunger, a mobile component of the injection device that triggers the injection operation, a needle shield, or any combination thereof, and wherein the sensor is configured to detect the movement of the at least one of the stopper of the plunger, the rod of the plunger, the mobile trigger, the mobile component, the needle shield, or any combination thereof.

3. The sensor assembly of claim 1, wherein the information associated with the movement of the component of the injection device includes at least one of the following: a start time of the injection operation, an end time of the injection operation, a duration of the injection operation, a speed of the injection operation, a back pressure profile of the injection operation, a time of a needle shield deployment, a measured internal noise or vibration of the injection device, a measured injection force, or any combination thereof.

4. The sensor assembly of claim 1, wherein the housing includes one or more processors programmed and/or configured to determine, based on sensor data received from the sensor, the information associated with the movement of the component of the injection device.

5. The sensor assembly of claim 1, wherein the housing includes a user feedback device, and wherein the user feedback device includes at least one of the following: a display, a light-emitting diode (LED), an audio output device, or any combination thereof.

6. The sensor assembly of claim 1, wherein the housing includes a battery configured to power the sensor.

7. The sensor assembly of claim 1, wherein the wireless communication device includes a short range wireless communication device.

8. The sensor assembly of claim 1, wherein the housing includes a memory.

9. The sensor assembly of claim 1, wherein the injection device includes one of the following: an auto injector, a pen injector, a wearable injector, a safety syringe device, a pre-filled syringe, and a passive needle guard.

10. The sensor assembly of claim 1, wherein the mechanical interface is configured to index a position of the sensor relative to a drug inspection window of the injection device, and wherein the sensor is calibrated according to the indexed position of the sensor relative to the drug inspection window of the injection device.

11. The sensor assembly of claim 1, wherein the housing is removably attached to the mechanical interface.

12. The sensor assembly of claim 1, wherein the housing is integrated with the mechanical interface.

13. The sensor assembly of claim 1, wherein the mechanical interface includes a base plate configured to be attached to the exterior surface of the injection device.

14. The sensor assembly of claim 1, wherein the mechanical interface includes the exterior surface of the injection device.

15. The sensor assembly of claim 1, wherein the mechanical interface includes an adhesive.

16. The sensor assembly of claim 1, wherein the mechanical interface includes an adhesive label.

17. The sensor assembly of claim 1, wherein the mechanical interface includes a ring or sleeve configured to fit around the exterior surface of the injection device, and wherein the ring or sleeve includes at least one of an elastomeric material and a heat-shrinkable material.

18. The sensor assembly of claim 1, wherein the mechanical interface includes a rigid deformable clip configured to clip onto the exterior surface of the injection device.

19. The sensor assembly of claim 1, wherein the sensor includes an optical sensor.

20. The sensor assembly of claim 19, wherein, when the housing is attached to the injection device via the mechanical interface, the optical sensor is positioned to detect the movement of the component of the injection device through a drug inspection window of the injection device.

21. The sensor assembly of claim 20, wherein the optical sensor includes an array of photodetector and LED pairs.

22. The sensor assembly of claim 20, wherein the optical sensor includes a single photodetector and LED pair, and wherein the component of the injection device includes a printed or gradated pattern visible through the drug inspection window of the injection device.

23. The sensor assembly of claim 19, wherein, when the housing is attached to the injection device via the mechanical interface, the housing partially overlaps a drug inspection window of the injection device.

24. The sensor assembly of claim 19, wherein the injection device includes a plurality of drug inspection windows, and wherein, when the housing is attached to the injection device via the mechanical interface, the housing fully overlaps one of the plurality of drug inspection windows.

25. The sensor assembly of claim 1, wherein the sensor includes an acoustic or vibration sensor.

26. The sensor assembly of claim 25, wherein, when the housing is attached to the injection device via the mechanical interface, the acoustic or vibration sensor is positioned to measure an internal noise or vibration of the injection device to detect the movement of the component of the injection device.

27. The sensor assembly of claim 25, further comprising:
one or more processors programmed and/or configured to compare a signal received from the acoustic sensor or the vibration sensor to one or more reference signals to determine at least one of the following: a start time of the injection operation, a duration of the injection operation, an end time of the injection operation, a speed of the injection operation, a back pressure profile of the injection operation, a time of a needle shield deployment, a measured internal noise or vibration of the injection device, a measured injection force, or any combination thereof.

28. The sensor assembly of claim 1, wherein the sensor includes a magnetic sensor.

29. The sensor assembly of claim 28, wherein the component of the injection device includes a magnet, and wherein, when the housing is attached to the injection device via the mechanical interface, the magnetic sensor is positioned to measure a magnetic field modification and/or a magnetic evolution created by the magnet to detect the movement of the component of the injection device.

30. The sensor assembly of claim 29, wherein, when the housing is attached to the injection device via the mechanical interface, the magnetic sensor is positioned at a center of a range of motion of the magnet.

31. The sensor assembly of claim 29, wherein the magnet includes a printed or gradated magnetic pattern or scale.

32. A system comprising:
an injection device;
the sensor assembly according to any of claims 1-31.
